(19) Europäisches Patentamt
European Patent Office
Office européen des brevets

(11) Numéro de publication: **0 390 673 A1**

(12) **DEMANDE DE BREVET EUROPEEN**

(21) Numéro de dépôt: **90400834.9**

(51) Int. Cl.5: **C07D 263/58, A61K 31/42, C07D 413/06**

(22) Date de dépôt: **28.03.90**

(30) Priorité: **30.03.89 FR 8904129**

(43) Date de publication de la demande: **03.10.90 Bulletin 90/40**

(84) Etats contractants désignés: **AT BE CH DE DK ES FR GB GR IT LI LU NL SE**

(71) Demandeur: **ADIR ET COMPAGNIE**
**22, rue Garnier**
**F-92201 Neuilly sur Seine(FR)**

(72) Inventeur: **Lesieur, Daniel**
**20 rue de Verdun**
**F-59147 Gondecourt(FR)**
Inventeur: **Aichaoui, Hocine**
**160 rue de la Paix Tour Kennedy**
**F-59120 Loos(FR)**
Inventeur: **Lespagnol, Charles**
**115 avenue Pasteur**
**F-59130 Lambersart(FR)**
Inventeur: **Bonnet, Jacqueline**
**19 rue Charcot**
**F-75013 Paris(FR)**

(54) **Nouveaux dérivés d'acyl-5 benzoxazolinone, leurs procédés de préparation et les compositions pharmaceutiques qui les contiennent.**

(57) Composés de formule générale (I) :

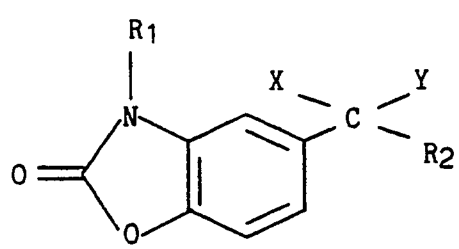

(I)

dans laquelle :
$R_1$ représente un atome d'hydrogène ou un groupement alkyle inférieur,
$R_2$ représente un alkyle inférieur, substitué ou non, un aryle substitué ou non, un groupement thiényle, furyle ou pyrrolyle,
X représente un atome d'hydrogène,
Y représente un atome d'hydrogène, ou un hydroxyle,
ou bien X et Y représentent ensemble un atome d'oxygène, à la condition que dans ce cas $R_2$ ne représente pas un radical alkyle inférieur non substitué,
leurs énantiomères, diastéréoisomères et épimères.
Médicaments.

EP 0 390 673 A1

# NOUVEAUX DERIVES D'ACYL - 5 BENZOXAZOLINONE, LEURS PROCEDES DE PREPARATION ET LES COMPOSITIONS PHARMACEUTIQUES QUI LES CONTIENNENT

La présente invention concerne de nouveaux dérivés de la benzoxazolinone, leurs préparations et les compositions pharmaceutiques qui les contiennent.

De nombreux dérivés de la benzoxazolinone ont été décrits en thérapeutique comme possédant des activités pharmacologiques très variées.

Le brevet Fr 73.23280 décrit notamment les acyl-6 benzoxazolinones, en tant qu'analgésiques.

Le brevet Fr 80.20861 décrit des (amino-2 hydroxy-1 éthyl)-6 benzoxazolinones et des amino acétyl-6 benzoxazolinones utilisables en tant qu'antihypertenseurs. Enfin, le brevet Fr.82.19812 décrit des (amino-2 éthyl)-6 benzoxazolinones possédant une activité thérapeutique de type psycholeptique.

La demanderesse a maintenant découvert des dérivés benzoxazolinoniques doués d'une activité analgésique dépourvue d'activité anti-inflammatoire d'un niveau nettement plus intéressant que celui des dérivés décrits dans les brevets Fr 73.23280 et Fr 80.20861. Les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thromboxane A2...) ; il s'ensuit donc de multiples effets secondaires dont les plus connus sont : attaque de la muqueuse gastrique avec possibilité d'ulcères, inhibition de l'agrégation plaquettaire avec troubles de la coagulation. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent pas sur les médiateurs de l'inflammation et sont donc dépourvus des effets secondaires mentionnés précédemment. Cette caractéristique, jointe à leur absence totale de toxicité et à leur haut niveau d'activité rend certains composés de la présente invention utilisables en tant qu'analgésiques d'une façon beaucoup plus sûre et sans les restrictions d'usage habituellement connues pour la grande majorité de ces produits.

Plus spécifiquement, l'invention concerne les dérivés de formule générale (I) :

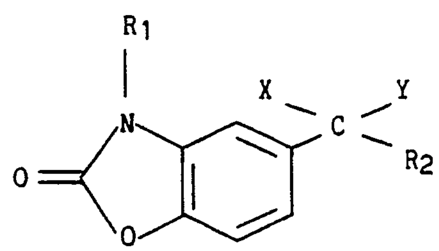

(I)

dans laquelle :

$R_1$ représente un atome d'hydrogène ou un groupement alkyle inférieur,

$R_2$ représente :

. un radical alkyle inférieur éventuellement substitué par :

- un ou plusieurs atomes d'halogène,
- un groupement aryle ou hétéroaryle,

. un groupement aryle éventuellement substitué par :

- un ou plusieurs atomes d'halogène,
- un ou plusieurs groupements alkyles inférieurs éventuellement substitués par un ou plusieurs atomes d'halogène,
- un ou plusieurs groupements alkoxy inférieurs,

. un groupement thiényle-2, éventuellement substitué par un ou plusieurs groupements alkyles inférieurs linéaires ou ramifiés,

. un groupement furyle-2, éventuellement substitué par un ou plusieurs groupements alkyles inférieurs linéaires ou ramifiés,

. un groupement pyrrolyle-2, éventuellement substitué par un ou plusieurs groupements alkyles inférieurs linéaires ou ramifiés,

X représente un atome d'hydrogène,

Y représente un atome d'hydrogène, un groupement hydroxyle,
ou bien,
X et Y représentent ensemble un atome d'oxygène, à la condition que dans ce cas R2 ne représente pas un groupement alkyle inférieur non substitué,
le terme inférieur indiquant que les groupements ainsi qualifiés comportent 1 à 6 atomes de carbone,
leurs énantiomères, diastéréoisomères et épimères.

L'invention s'étend aussi au procédé d'obtention des composés de formule générale (I), caractérisé en ce que l'on utilise comme matière première l'amino-2 phénol de formule (II) :

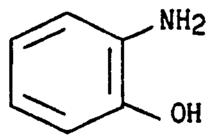

(II)

sur lequel on fait réagir l'anhydride acétique pour conduire à l'acétylamino-2 phénol de formule (III) :

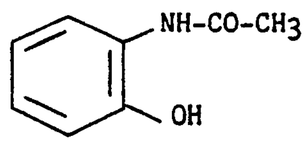

(III)

que l'on soumet à l'action d'un chlorure d'acide de formule (IV) :
**$R_2$ - CO - Cl (IV)**
dans laquelle $R_2$ a la même signification que dans la formule (I),
ou bien de l'anhydride d'acide correspondant,
dans les conditions classiques de la réaction de Friedel-Crafts en utilisant préférentiellement le chlorure d'aluminium en présence de diméthylformamide selon les conditions de THYES et Coll. (J. Med. Chem., 1983, 26, 6, 800-807) pour obtenir un dérivé de formule générale (V) :

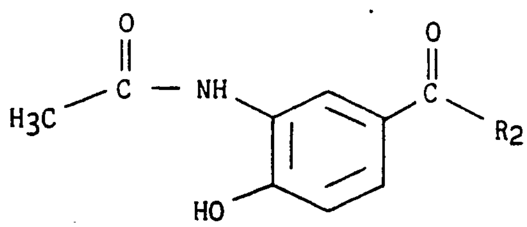

(V)

dans laquelle $R_2$ a la même signification que dans la formule (I),
qui est alors hydrolysé préférentiellement en milieu acide à reflux du milieu réactionnel pour conduire après évaporation puis reprise en milieu acide en présence d'urée à une température comprise entre la température ambiante et la température d'ébullition du milieu ou bien soumis à l'action du phosgène, à un dérivé de formule générale (I/a) :

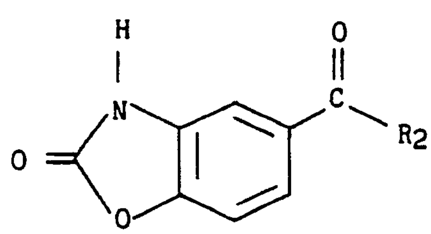

(I/a)

cas particulier des dérivés de formule (I) dans laquelle :
$R_2$ a la même signification que dans la formule (I),
$R_1$ est un atome d'hydrogène,
X et Y représentent ensemble un atome d'oxygène,

qui, si on le souhaite, est soumis, en fonction de la structure des dérivés de formule (I) que l'on souhaite obtenir, à l'action d'un agent classique d'alkylation comme par exemple un dialkylsulfate pour conduire à un dérivé de formule (I/b) :

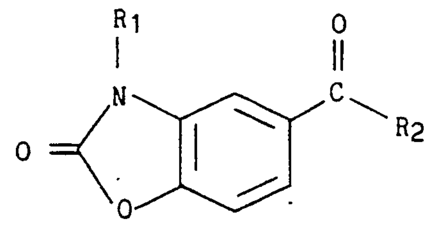

(I/b)

cas particulier des dérivés de formule (I) dans laquelle :
$R_1$ représente un groupement alkyle inférieur,
$R_2$ a la même signification que dans la formule (I),
X et Y représentent ensemble un atome d'oxygène,
dérivé de formule (I/a) ou (I/b), selon le dérivé de formule (I) que l'on souhaite obtenir, qui peut être soumis, si on le désire,
ou bien
à un agent d'hydrogénation préférentiellement choisi parmi un hydrure mixte de métal alcalin comme par exemple, le borohydrure de sodium, préférentiellement en présence d'alcool aliphatique inférieur, pour conduire à un dérivé de formule (I/c) :

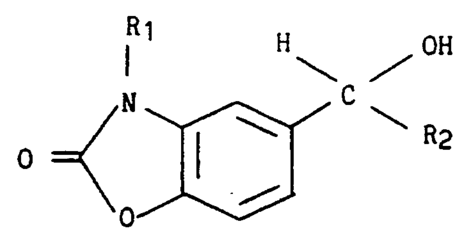

(I/c)

cas particulier des dérivés de formule (I) dans laquelle :
$R_1$ et $R_2$ ont la même signification que dans la formule (I),
X représente un atome d'hydrogène,
Y représente un groupement hydroxyle,
dont on sépare les isomères, si on le souhaite, par une technique classique de séparation,
ou bien
à une réduction par un trialkylsilane en milieu acide selon les conditions décrites par West et Coll. (J. Org. Chem., 1973, 38, (15), 2675-2681) pour conduire à un dérivé de formule (I/d) :

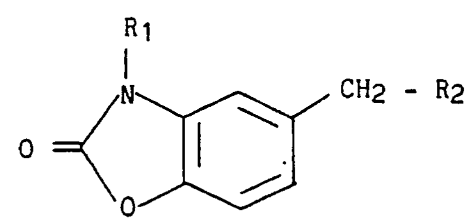

(I/d)

cas particulier des dérivés de formule (I) dans laquelle :
$R_1$ et $R_2$ ont la même signification que dans la formule (I),
X et Y représentent chacun un atome d'hydrogène,
dérivés de formule (I) que l'on purifie, si on le désire, par recristallisation, chromatographie ou toute autre technique classique de purification.

Les composés de formule (I) possèdent des propriétés pharmacologiques intéressantes.

En particulier ces dérivés ont révélé une activité analgésique intéressante.

L'étude pharmacologique des dérivés de l'invention a en effet montré qu'ils étaient peu toxiques, doués d'une activité analgésique pure et donc dépourvus d'inconvénients inhérents à la plupart des composés non morphiniques présentant cette activité, (action ulcérigène sur les muqueuses, perturbation de la coagulation...). Ce spectre d'activité rend donc les composés de la présente invention intéressants dans un certain nombre d'indications telles que algies rhumatismales, névralgies, lombosciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant les produits de formule (I), seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique ou des traitements éventuellement associés et s'échelonne entre 1 centigramme et 4 grammes par 24 heures.

Les exemples suivants illustrent l'invention et ne la limitent en aucune façon.

**EXEMPLE 1 : BENZOYL-5 BENZOXAZOLINONE**

**STADE A : ACETYLAMINO-2 PHENOL**

Dans une fiole rodée contenant 10 g (0,09 mole) d'amino-2 phénol en suspension dans 60 ml d'eau, ajouter progressivement et sous agitation magnétique 13,2 ml (0,14 mole) d'anhydride acétique et laisser sous agitation pendant 12 heures à température ambiante.
Essorer le précipité formé, laver à l'eau et sécher.
Recristalliser dans de l'éthanol.
Rendement : 92 %
Point de fusion : 210-211 °C
Infrarouge : ν CO : 1650 $cm^{-1}$

**STADE B : ACETYLAMINO-2 BENZOYL-4 PHENOL**

Dans une fiole rodée contenant 46,7 g (0,35 mole) de chlorure d'aluminium anhydre, introduire goutte à goutte et sous agitation 7,5 ml de diméthylformamide (0,98 mole).
Munir la fiole d'un réfrigérant à reflux et porter dans un bain d'huile à une température voisine de 35 °C.
Introduire 0,05 mole d'acétylamino-2 phénol préparé au stade A et 0,07 mole de chlorure d'acide benzoïque.
Chauffer à une température voisine de 90 °C pendant environ 5 heures.
Après refroidissement, verser le mélange réactionnel dans de la glace, acidifier par de l'acide chlorhydrique concentré et laisser sous agitation pendant une heure.
Essorer le précipité obtenu, laver à l'eau et sécher.
Recristalliser dans de l'éthanol.
Rendement : 75 %
Point de fusion : 193-194 °C
Infrarouge : ν CO : 1650 $cm^{-1}$ et 1640 $cm^{-1}$

| Microanalyse élémentaire : | | | |
|---|---|---|---|
| | C % | H % | N % |
| Calculé<br>Trouvé | 70,58<br>71,33 | 5,13<br>5,13 | 5,48<br>5,42 |

**STADE C : BENZOYL-5 BENZOXAZOLINONE**

Dans un ballon équipé d'un réfrigérant, mettre 0,46 mole d'acétylamino-2 benzoyl-4 phénol préparé au stade B, dans 45 ml d'acide chlorhydrique concentré. Porter à reflux pendant 30 minutes. Après refroidissement, évaporer à sec au bain-marie sous vide. Placer alors le résidu dans 5 ml d'acide chlorhydrique concentré en présence de 0,10 mole d'urée et chauffer à une température voisine de 145 °C pendant environ 4 heures.
Après refroidissement, diluer par de l'eau et agiter pendant 30 minutes.
Essorer le précipité obtenu, laver à l'eau et sécher.
Recristalliser dans de l'éthanol.
Rendement : 76 %
Point de fusion : 169-170 °C
Infrarouge : ν CO (carbamate) : 1760 $cm^{-1}$
ν CO (cétone) : 1655 $cm^{-1}$

| Microanalyse élémentaire : | | | |
|---|---|---|---|
| | C % | H % | N % |
| Calculé<br>Trouvé | 70,28<br>70,43 | 3,79<br>3,73 | 5,86<br>6,01 |

**EXEMPLE 2 : (CHLORO-4 BENZOYL)-5 BENZOXAZOLINONE**

**STADE A : ACETYLAMINO-2 PHENOL**

Préparé comme dans l'exemple 1, stade A.

**STADE B : ACETYLAMINO-2 (CHLORO-4 BENZOYL)-4 PHENOL**

Procéder comme dans le stade B de l'exemple 1, en remplaçant le chlorure d'acide benzoïque par le chlorure d'acide chloro-4 benzoïque et en maintenant le chauffage pendant environ 5 heures à une température voisine de 90 °C.
Recristalliser dans de l'éthanol.
Rendement : 74 %
Point de fusion : 259-260 °C
Infrarouge : ν CO : vers 1660 $cm^{-1}$

| Microanalyse élémentaire : | | | | |
|---|---|---|---|---|
| | C % | H % | N % | Cl % |
| Calculé | 62,18 | 4,17 | 4,83 | 12,25 |
| Trouvé | 61,96 | 4,24 | 4,79 | 12,06 |

**STADE C : (CHLORO-4 BENZOYL)-5 BENZOXAZOLINONE**

Procéder comme dans le stade C de l'exemple 1, en remplaçant l'acétylamino-2 benzoyl-4 phénol par l'acétylamino-2 (chloro-4 benzoyl)-4 phénol obtenu au stade B
Recristalliser dans de l'éthanol.
Rendement : 74 % Point de fusion : 215-216 °C
Infrarouge : ν CO (carbamate) : 1760 $cm^{-1}$
ν CO (cétone) : 1650 $cm^{-1}$

| Microanalyse élémentaire : | | | | |
|---|---|---|---|---|
| | C % | H % | N % | Cl % |
| Calculé | 61,43 | 2,94 | 5,11 | 12,96 |
| Trouvé | 61,20 | 2,24 | 5,02 | 12,79 |

**EXEMPLE 3 : (THENOYL-2)-5 BENZOXAZOLINONE**

**STADE A : ACETYLAMINO-2 PHENOL**

Préparé comme dans l'exemple 1, stade A.

**STADE B : ACETYLAMINO-2 (THENOYL-2)-4 PHENOL**

Procéder comme dans le stade B de l'exemple 1, en remplacant le chlorure d'acide benzoïque par le chlorure de l'acide thiophène-2 carboxylique et en maintenant le chauffage pendant environ 3 heures 30 minutes à une température voisine de 85 °C.
Recristalliser dans un mélange éthanol/eau (1/1).
Rendement : 72 %
Point de fusion : 199-200 °C
Infrarouge : ν CO : vers 1650 $cm^{-1}$

| Microanalyse élémentaire : | | | | |
|---|---|---|---|---|
| | C % | H % | N % | S % |
| Calculé | 59,76 | 4,58 | 5,36 | 12,27 |
| Trouvé | 59,68 | 4,34 | 5,31 | 12,25 |

**STADE C : (THENOYL-2)-5 BENZOXAZOLINONE**

Procéder comme dans le stade C de l'exemple 1, en remplaçant l'acétylamino-2 benzoyl-4 phénol par l'acétylamino-2 (thénoyl-2)-4 phénol obtenu au stade B.
Recristalliser dans un mélange éthanol/eau (1/1).
Rendement : 71 %
Point de fusion : 215-217 °C
Infrarouge : ν CO (carbamate) : vers 1800 $cm^{-1}$
ν CO (cétone) : 1630 $cm^{-1}$

| Microanalyse élémentaire : | | | | |
|---|---|---|---|---|
| | C % | H % | N % | S % |
| Calculé<br>Trouvé | 58,76<br>58,64 | 2,88<br>3,05 | 5,71<br>5,79 | 13,07<br>12,82 |

**EXEMPLE 4 : (TRIFLUOROMETHYL-3 BENZOYL)-5 BENZOXAZOLINONE**

En procédant comme dans l'exemple 1, mais en remplaçant le chlorure d'acide benzoïque par le chlorure d'acide trifluorométhyl-3 benzoïque (stade B), on obtient le produit attendu.

**EXEMPLE 5 : (FLUORO-2 BENZOYL)-5 BENZOXAZOLINONE**

En procédant comme dans l'exemple 1, mais en remplaçant le chlorure d'acide benzoïque par le chlorure d'acide fluoro-2 benzoïque, on obtient le produit attendu.

**EXEMPLE 6 : VALERYL-5 BENZOXAZOLINONE**

En procédant comme dans l'exemple 1, mais en remplaçant le chlorure d'acide benzoïque par le chlorure d'acide valérique, on obtient le produit attendu.

**EXEMPLE 7 : (FUROYL-2)-5 BENZOXAZOLINONE**

En procédant comme dans l'exemple 1, mais en remplaçant le chlorure d'acide benzoïque par le chlorure de l'acide furanne-2 carboxylique, on obtient le produit attendu.

**EXEMPLE 8 : (PYRROLYL-2 CARBONYL)-5 BENZOXAZOLINONE**

En procédant comme dans l'exemple 1, mais en remplaçant le chlorure d'acide benzoïque par le chlorure d'acide pyrrole-2 carboxylique, on obtient le produit attendu.

**EXEMPLE 9 : METHYL-3 BENZOYL-5 BENZOXAZOLINONE**

Dans une fiole rodée, dissoudre dans 5,2 ml de solution aqueuse de soude à 10 %, 0,013 mole de benzoyl-5 benzoxazolinone obtenue dans l'exemple 1.
Après dissolution, diluer par de l'eau et introduire goutte à goutte, à 0 °C, sous agitation, 0,013 mole de sulfate de diméthyle. Maintenir sous agitation à cette température pendant 2 heures.
Essorer le précipité obtenu, laver à l'eau et sécher.
Recristalliser dans de l'éthanol.
Rendement : 95 %
Point de fusion : 151-152 °C

Infrarouge : ν CO (carbamate) : 1770 $cm^{-1}$
ν CO (cétone) : 1645 $cm^{-1}$

| Microanalyse élémentaire : | | | |
|---|---|---|---|
| | C % | H % | N % |
| Calculé | 71,13 | 4,38 | 5,53 |
| Trouvé | 71,11 | 4,99 | 5,55 |

**EXEMPLE 10 : METHYL-3 (CHLORO-4 BENZOYL)-5 BENZOXAZOLINONE**

Procéder comme dans l'exemple 9, mais en remplaçant la benzoyl-5 benzoxazolinone par la (chloro-4 benzoyl)-5 benzoxazolinone obtenue dans l'exemple 2.
Recristalliser dans de l'éthanol.
Rendement : 93 %
Point de fusion : 161-162 °C
Infrarouge : ν CO (carbamate) : 1770 $cm^{-1}$
ν CO (cétone) : 1645 $cm^{-1}$

| Microanalyse élémentaire : | | | | |
|---|---|---|---|---|
| | C % | H % | N % | Cl % |
| Calculé | 62,61 | 3,50 | 4,86 | 12,33 |
| Trouvé | 62,32 | 3,50 | 4,79 | 12,28 |

**EXEMPLE 11 : METHYL-3 (THENOYL-2)-5 BENZOXAZOLINONE**

Procéder comme dans l'exemple 9, mais en remplaçant la benzoyl-5 benzoxazolinone par la (thénoyl-2)-5 benzoxazolinone obtenue dans l'exemple 3.
Recristallisation dans de l'éthanol.
Rendement : 92 %
Point de fusion : 172-173 °C
Infrarouge : ν CO (carbamate) : 1780 $cm^{-1}$
ν CO (cétone) 1635 $cm^{-1}$

| Microanalyse élémentaire : | | | | |
|---|---|---|---|---|
| | C % | H % | N % | S % |
| Calculé | 60,22 | 3,49 | 5,40 | 12,36 |
| Trouvé | 60,36 | 3,05 | 5,79 | 12,66 |

**EXEMPLE 12 : METHYL-3 (TRIFLUOROMETHYL-3 BENZOYL)-5 BENZOXAZOLINONE**

En procédant comme dans l'exemple 9, mais en remplaçant la benzoyl-5 benzoxazolinone par la (trifluorométhyl-3 benzoyl)-5 benzoxazolinone obtenue dans l'exemple 4, on obtient le produit attendu.

**EXEMPLE 13 : METHYL-3 (FLUORO-2 BENZOYL)-5 BENZOXAZOLINONE**

En procédant comme dans l'exemple 9, mais en remplaçant la benzoyl-5 benzoxazolinone par la (fluoro-2 benzoyl)-5 benzoxazolinone obtenue dans l'exemple 6, on obtient le produit attendu.

**EXEMPLE 14 : METHYL-3 VALERYL-5 BENZOXAZOLINONE**

En procédant comme dans l'exemple 9, mais en remplaçant la benzoyl-5 benzoxazolinone par la valéryl-5 benzoxazolinone obtenue dans l'exemple 7, on obtient le produit attendu.

**EXEMPLE 15 : METHYL-3 (FUROYL-2)-5 BENZOXAZOLINONE**

En procédant comme dans l'exemple 9, mais en remplaçant la benzoyl-5 benzoxazolinone par la (furoyl-2)-5 benzoxazolinone, on obtient le produit attendu.

**EXEMPLE 16 : METHYL-3 (PYRROLYL-2 CARBONYL)-5 BENZOXAZOLINONE**

En procédant comme dans l'exemple 9, mais en remplaçant la benzoyl-5 benzoxazolinone par la (pyrrolyl-2 carbonyl)-5 benzoxazolinone, on obtient le produit attendu.

**EXEMPLE 17 : METHYL-3 (HYDROXY-1 PHENYL-1 METHYL)-5 BENZOXAZOLINONE**

Dans une fiole de 250 $cm^3$, dissoudre dans 200 $cm^3$ de méthanol sous agitation magnétique, 0,01 mole de méthyl-3 benzoyl-5 benzoxazolinone préparée dans l'exemple 9. Ajouter très lentement et sous agitation 0,02 mole de borohydrure de sodium. Maintenir l'agitation 4 heures.
Evaporer le milieu réactionnel au bain-marie sous vide. Reprendre le résidu par de l'eau et extraire plusieurs fois au chloroforme. Sécher sur chlorure de calcium. Filtrer, évaporer à sec au bain-marie et recristalliser le résidu.

**EXEMPLE 18 : METHYL-3 BENZYL-5 BENZOXAZOLINONE**

Dans une fiole rodée, dissoudre 0,02 mole de méthyl-3benzoyl-5 benzoxazolinone préparée dans l'exemple 9 dans 0,2 mole d'acide trifluoroacétique.
Ajouter goutte à goutte et en refroidissant 0,044 mole de triéthylsilane. Adapter une garde à chlorure de calcium et poursuivre l'agitation pendant 72 heures. Verser alors le milieu réactionnel dans de l'eau glacée, essorer le précipité, sécher et recristalliser.

**EXEMPLE 19 : METHYL-3 ETHYL-5 BENZOXAZOLINONE**

En procédant comme dans l'exemple 18 en remplaçant la méthyl-3 benzoyl-5 benzoxazolinone par la méthyl-3 acétyl-5 benzoxazolinone

**EXEMPLE 20 : CHLOROACETYL-5 BENZOXAZOLINONE**

En remplaçant dans l'exemple 1, stade B le chlorure de l'acide benzoïque par le chlorure de chloracétyle, on obtient le produit du titre.

**EXEMPLE 21 : PHENYLACETYL-5 BENZOXAZOLINONE**

En remplaçant dans l'exemple 1, stade B le chlorure de l'acide benzoïque par le chlorure de

phénacétyle, on obtient le produit en titre.

**EXEMPLE 22 : (METHYL-3 PHENOYL-2)-5 BENZOXAZOLINONE**

En procédant comme dans l'exemple 3 mais en remplaçant le chlorure de l'acide méthyl-3 thiophène-2 carboxylique, on obtient le produit du titre.

**EXEMPLE 23 : 0 ANISOYL-5 BENZOXAZOLINONE**

En remplaçant dans l'exemple 1, stade B le chlorure de l'acide benzoïque par le chlorure de l'acide o anisique, on obtient le produit du titre

**ETUDE PHARMACOLOGIQUE DES DERIVES DE L'INVENTION**

**EXEMPLE 24 : ETUDE DE LA TOXICITE AIGUE**

La toxicité aiguë a été appréciée après administration orale à des lots de 8 souris (26±2 grammes) d'une dose de 1000 $mg.kg^{-1}$. Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose de 1000 $mg.kg^{-1}$. On ne constate pas de troubles après administration de cette dose.

**EXEMPLE 25 : ETUDE DE L'ACTIVITE ANALGESIQUE**

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50%, a été déterminée pour chaque produit.

Il est apparu que certains composés de l'invention possèdent une activité analgésique très intéressante. Ainsi, l'$ED_{50}$ du composé de l'exemple 1 est voisine de 5 $mg.kg^{-1}$.

A titre de comparaison l'administration d'une dose de 100 $mg.kg^{-1}$ des dérivés du brevet Fr 73.23280 provoquait un pourcentage d'analgésie -dans un test comparable - de l'ordre de 25 à 60 % et le composé du brevet Fr 80.20861 dont l'activité analgésique est la plus intéressante avait dans ce même test de Siegmund une $ED_{50}$ de 9 $mg.kg^{-1}$ soit environ 2 fois supérieure à celle du produit le plus intéressant de la présente invention.

**EXEMPLE 26 : ETUDE DE L'ACTIVITE ANTI-INFLAMMATOIRE**

Le potentiel anti-inflammatoire des composés à été recherché sur un modèle d'inflammation aiguë provoquée par injection sous-cutanée d'une solution de carragénine au niveau de la patte postérieure de rat, selon une technique inspirée de la méthode de WINTERCH E.A. RISLEY, G.N. NUSS - (Proc. Soc. Exp. Med. 111, 554, 1962). Les rats (100-120 g), randomisés en lots de 8, ont été traités (y compris les témoins qui reçoivent l'excipient) 1 heure avant l'injection locale d'une suspension à 0,5% de carragénine (type IV, Sigma ; 0,1 ml de rat). L'oedème est déterminé 3 heures après l'injection, par mesure pléthysmométrique (pléthysmomètre à eau UGO BASILE) du volume de chacune des pattes postérieures (oedème = volume de la patte enflammée diminué du volume de la patte non enflammée).

Il apparaît que les produits de l'invention n'ont aucune activité sur ce test. En comparaison, les produits du brevet Fr 73.23280 possèdent une activité anti-inflammatoire.

**EXEMPLE 27 : COMPOSITION PHARMACEUTIQUE : COMPRIME**

Comprimés dosés à 20 mg de benzoyl-5 benzoxazolinone

| Formule de préparation pour 1000 comprimés. | |
|---|---|
| Benzoyl-5 benzoxazolinone .... | 20 g |
| Amidon de blé .... | 15 g |
| Amidon de maïs .... | 15 g |
| Lactose .... | 65 g |
| Stéarate de magnésium .... | 2 g |
| Silice .... | 1 g |
| Hydroxypropylcellulose .... | 2 g |

**Revendications**

1/ Composés de formule générale (I) :

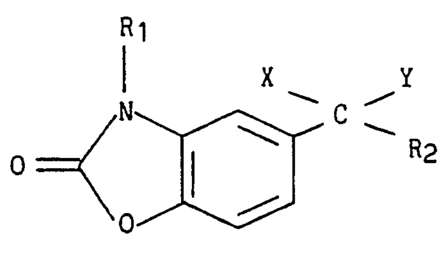

(I)

dans laquelle :
$R_1$ représente un atome d'hydrogène ou un groupement alkyle inférieur,
$R_2$ représente :
. un radical alkyle inférieur éventuellement substitué par :
- un ou plusieurs atomes d'halogène,
- un groupement aryle ou hétéroaryle,
. un groupement aryle éventuellement substitué par :
- un ou plusieurs atomes d'halogène,
- un ou plusieurs groupements alkyles inférieurs éventuellement substitués par un ou plusieurs atomes d'halogène,
- un ou plusieurs groupements alkoxy inférieurs,
. un groupement thiényle-2, éventuellement substitué par un ou plusieurs groupements alkyles inférieurs linéaires ou ramifiés,
. un groupement furyle-2, éventuellement substitué par un ou plusieurs groupements alkyles inférieurs linéaires ou ramifiés,
. un groupement pyrrolyle-2, éventuellement substitué par un ou plusieurs groupements alkyles inférieurs linéaires ou ramifiés.
X représente un atome d'hydrogène,
Y un représente un atome d'hydrogène, ou un groupement hydroxyle,

ou bien, X et Y représentent ensemble un atome d'oxygène, à la condition que dans ce cas $R_2$ ne représente pas un groupement alkyle inférieur non substitué
le terme inférieur indiquant que les groupements ainsi qualifiés comportent 1 à 6 atomes de carbone,
leurs énantiomères, diastéréoisomères et épimères.

2/ Composés selon la revendication 1, dans lesquels X et Y représentent ensemble un atome d'oxygène.

3/ Composés selon la revendication 1, dans lesquels $R_1$ représente un atome d'hydrogène.

4/ Composés selon la revendication 1, dans lesquels $R_1$ représente un groupement méthyle.

5/ Composés selon la revendication 1, dans lesquels $R_2$ représente un groupement aryle.

6/ Composé selon l'une des revendications 1, 2 et 5 qui est la benzoyl-5 benzoxazolinone.

7/ Composé selon l'une des revendications 1, 2 et 5 que est la méthyl-3 benzoyl-5 benzoxazolinone.

8/ Procédé de préparation des composés de formule générale (I) caractérisé en ce que l'on utilise comme matière première l'amino-2 phénol de formule (II) :

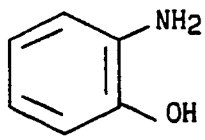

(II)

sur lequel on fait réagir l'anhydride acétique pour conduire à l'acétylamino-2 phénol de formule (III) :

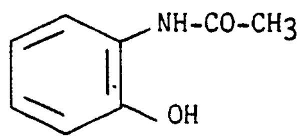

(III)

que l'on soumet à l'action d'un chlorure d'acide de formule (IV) :

**$R_2$ - CO - Cl (IV)**

dans laquelle $R_2$ a la même signification que dans la formule (I),
ou bien de l'anhydride d'acide correspondant,
préférentiellement en présence de chlorure d'aluminium dans le diméthylformamide, pour obtenir un dérivé de formule générale (V) :

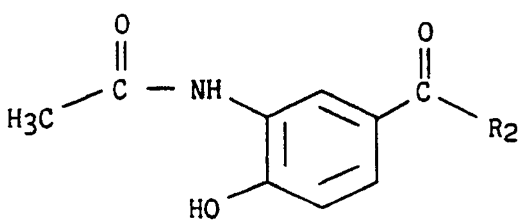

(V)

dans laquelle $R_2$ a la même signification que dans la formule (I),
qui est alors hydrolysé préférentiellement en milieu acide à reflux du milieu réactionnel pour conduire après évaporation puis reprise en milieu acide en présence d'urée à une température comprise entre la température ambiante et la température d'ébullition du milieu ou bien soumis à l'action phosgène, à un dérivé de formule générale (I/a) :

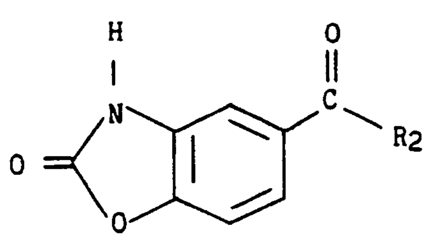

(I/a)

cas particulier des dérivés de formule (I) dans laquelle :
$R_1$ est un atome d'hydrogène,
$R_2$ a la même signification que dans la formule (I),
X et Y représentent ensemble un atome d'oxygène,
qui, si on le souhaite, est soumis en fonction de la structure des dérivés de formule (I) que l'on souhaite obtenir, à l'action d'un agent classique d'alkylation comme par exemple un dialkylsulfate pour conduire à un dérivé de formule (I/b) :

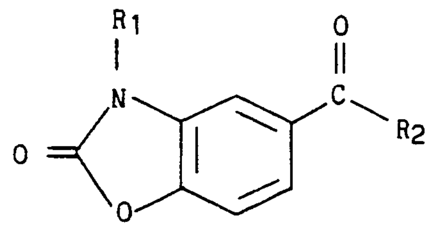

(I/b)

cas particulier des dérivés de formule (I) dans laquelle :
$R_1$ représente un groupement alkyle inférieur,
$R_2$ a la même signification que dans la formule (I),
X et Y représentent ensemble un atome d'oxygène,
dérivé de formule (I/a) ou (I/b), selon le dérivé de formule (I) que l'on souhaite obtenir, qui peut être soumis :
ou bien
à un hydrure mixte de métal alcalin comme par exemple le borohydrure de sodium, préférentiellement en présence d'alcool aliphatique inférieur, pour conduire à un dérivé de formule (I/c) :

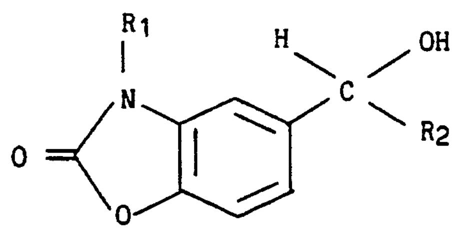

(I/c)

cas particulier des dérivés de formule (I) dans laquelle :
$R_1$ et $R_2$ ont la même signification que dans la formule (I),
X représente un atome d'hydrogène,
Y représente un groupement hydroxyle,
dont on sépare, si on le souhaite, les isomères, par une technique classique de séparation,
ou bien
à une réduction par un trialkylsilane en milieu acide pour conduire à un dérivé de formule (I/d) :

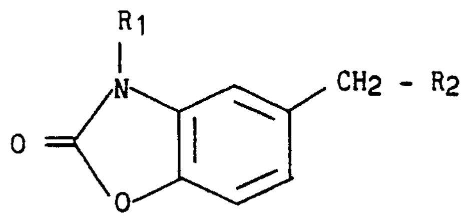

(I/d)

cas particulier des dérivés de formule (I) dans laquelle :
$R_1$ et $R_2$ ont la même signification que dans la formule (I),
X et Y représentent chacun un atome d'hydrogène,
dérivés de formule (I) que l'on purifie, si on le désire, par recristallisation, chromatographie ou toute autre technique classique de purification.

9/ Composition pharmaceutique contenant comme principe actif au moins un composé selon l'une des revendications 1 à 7 en combinaison avec un ou plusieurs excipients ou véhicules inertes, non toxiques, pharmaceutiquement acceptables.

10/ Composition pharmaceutique selon la revendication 9 contenant au moins un principe actif selon l'une des revendications 1 à 7 utilisables dans le traitement d'algies.

Revendications pour l'Etat contractant suivant : ES

1/ Procédé de préparation de composés de formule générale (I) :

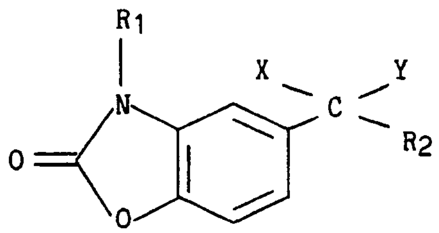

(I)

dans laquelle :

$R_1$ représente un atome d'hydrogène ou un groupement alkyle inférieur,

$R_2$ représente :

. un radical alkyle inférieur éventuellement substitué par :

- un ou plusieurs atomes d'halogène,
- un groupement aryle ou hétéroaryle,

. un groupement aryle éventuellement substitué par :

- un ou plusieurs atomes d'halogène,
- un ou plusieurs groupements alkyles inférieurs éventuellement substitués par un ou plusieurs atomes d'halogène,
- un ou plusieurs groupements alkoxy inférieurs,

. un groupement thiényle-2, éventuellement substitué par un ou plusieurs groupements alkyles inférieurs linéaires ou ramifiés,

. un groupement furyle-2, éventuellement substitué par un ou plusieurs groupements alkyles inférieurs linéaires ou ramifiés,

. un groupement pyrrolyle-2, éventuellement substitué par un ou plusieurs groupements alkyles inférieurs linéaires ou ramifiés.

X représente un atome d'hydrogène,

Y un représente un atome d'hydrogène, ou un groupement hydroxyle,

ou bien, X et Y représentent ensemble un atome d'oxygène, à la condition que dans ce cas $R_2$ ne représente pas un groupement alkyle inférieur non substitué

le terme inférieur indiquant que les groupements ainsi qualifiés comportent 1 à 6 atomes de carbone,

leurs énantiomères, diastéréoisomères et épimères,

caractérisé en ce que l'on utilise comme matière première l'amino-2 phénol de formule (II) :

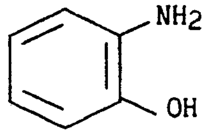

(II)

sur lequel on fait réagir l'anhydride acétique pour conduire à l'acétylamino-2 phénol de formule (III) :

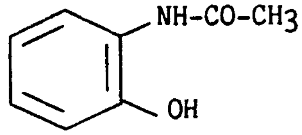

(III)

que l'on soumet à l'action d'un chlorure d'acide de formule (IV) :

**$R_2$ - CO - Cl (IV)**

dans laquelle $R_2$ a la même signification que dans la formule (I),
ou bien de l'anhydride d'acide correspondant,
préférentiellement en présence de chlorure d'aluminium dans le diméthylformamide, pour obtenir un dérivé de formule générale (V) :

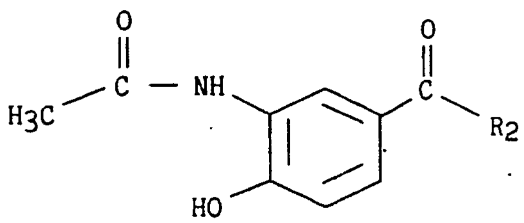

**(V)**

dans laquelle $R_2$ a la même signification que dans la formule (I),
qui est alors hydrolysé préférentiellement en milieu acide à reflux du milieu réactionnel pour conduire après évaporation puis reprise en milieu acide en présence d'urée à une température comprise entre la température ambiante et la température d'ébullition du milieu ou bien soumis à l'action phosgène, à un dérivé de formule générale (I/a) :

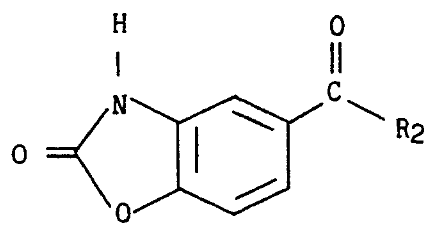

**(I/a)**

cas particulier des dérivés de formule (I) dans laquelle :
$R_1$ est un atome d'hydrogène,
$R_2$ a la même signification que dans la formule (I),
X et Y représentent ensemble un atome d'oxygène,
qui, si on le souhaite, est soumis en fonction de la structure des dérivés de formule (I) que l'on souhaite obtenir, à l'action d'un agent classique d'alkylation comme par exemple un dialkylsulfate pour conduire à un dérivé de formule (I/b) :

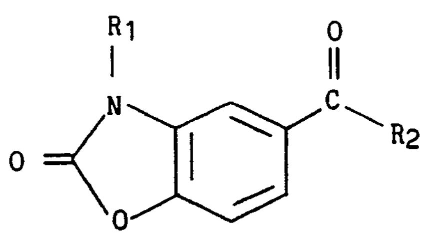

**(I/b)**

cas particulier des dérivés de formule (I) dans laquelle :
$R_1$ représente un groupement alkyle inférieur,
$R_2$ a la même signification que dans la formule (I),
X et Y représentent ensemble un atome d'oxygène,
dérivé de formule (I/a) ou (I/b), selon le dérivé de formule (I) que l'on souhaite obtenir, qui peut être soumis :
ou bien
à un hydrure mixte de métal alcalin comme par exemple le borohydrure de sodium, préférentiellement en présence d'alcool aliphatique inférieur, pour conduire à un dérivé de formule (I/c) :

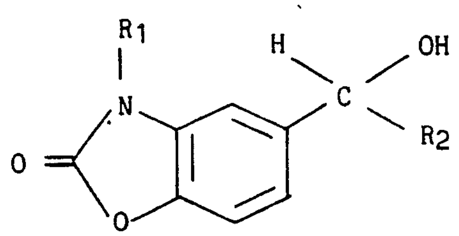

(I/c)

cas particulier des dérivés de formule (I) dans laquelle :
$R_1$ et $R_2$ ont la même signification que dans la formule (I),
X représente un atome d'hydrogène,
Y représente un groupement hydroxyle,
dont on sépare, si on le souhaite, les isomères, par une technique classique de séparation,
ou bien
à une réduction par un trialkylsilane en milieu acide pour conduire à un dérivé de formule (I/d) :

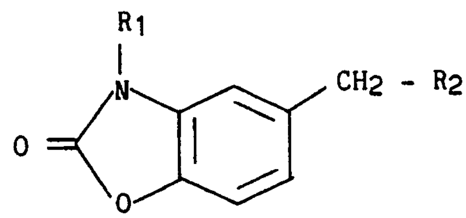

(I/d)

cas particulier des dérivés de formule (I) dans laquelle :
$R_1$ et $R_2$ ont la même signification que dans la formule (I),
X et Y représentent chacun un atome d'hydrogène,
dérivés de formule (I) que l'on purifie, si on le désire, par recristallisation, chromatographie ou toute autre technique classique de purification.

2/ Procédé selon la revendication 1, de préparation de composés de formule (I) dans lesquels X et Y représentent ensemble un atome d'oxygène.

3/ Procédé selon la revendication 1, de préparation de composés de formule (I) dans lesquels $R_1$ représente un atome d'hydrogène.

4/ Procédé selon la revendication 1, de préparation de composés de formule (I) dans lesquels $R_1$ représente un groupement méthyle.

5/ Procédé selon la revendication 1, de préparation de composés de formule (I) dans lesquels $R_2$ représente un groupement aryle.

6/ Procédé selon la revendication 1 de préparation du composé de formule (I) qui est la benzoyl-5 benzoxazolinone.

7/ Procédé selon la revendication 1 de préparation du composé de formule (I) qui est la méthyl-3 benzoyl-5 benzoxazolinone.

Revendications pour l'Etat contractant suivant: GR

1/ Procédé de préparation de composés de formule générale (I) :

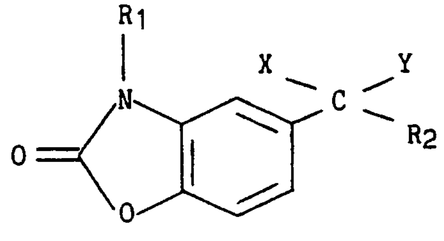

(I)

dans laquelle :

$R_1$ représente un atome d'hydrogène ou un groupement alkyle inférieur,
$R_2$ représente :
. un radical alkyle inférieur éventuellement substitué par :
- un ou plusieurs atomes d'halogène,
- un groupement aryle ou hétéroaryle, . un groupement aryle éventuellement substitué par :
- un ou plusieurs atomes d'halogène,
- un ou plusieurs groupements alkyles inférieurs éventuellement substitués par un ou plusieurs atomes d'halogène,
- un ou plusieurs groupements alkoxy inférieurs,
. un groupement thiényle-2, éventuellement substitué par un ou plusieurs groupements alkyles inférieurs linéaires ou ramifiés,
. un groupement furyle-2, éventuellement substitué par un ou plusieurs groupements alkyles inférieurs linéaires ou ramifiés,
. un groupement pyrrolyle-2, éventuellement substitué par un ou plusieurs groupements alkyles inférieurs linéaires ou ramifiés.
X représente un atome d'hydrogène,
Y un représente un atome d'hydrogène, ou un groupement hydroxyle,
ou bien, X et Y représentent ensemble un atome d'oxygène, à la condition que dans ce cas $R_2$ ne représente pas un groupement alkyle inférieur non substitué
le terme inférieur indiquant que les groupements ainsi qualifés comportent 1 à 6 atomes de carbone,
leurs énantiomères, diastéréoisomères et épimères,
caractérisé en ce que l'on utilise comme matière première l'amino-2 phénol de formule (II) :

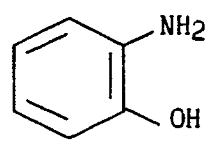

(II)

sur lequel on fait réagir l'anhydride acétique pour conduire à l'acétylamino-2 phénol de formule (III) :

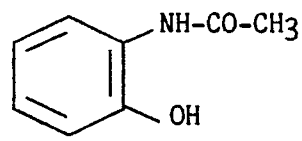

(III)

que l'on soumet à l'action d'un chlorure d'acide de formule (IV) :
**$R_2$ - CO - Cl (IV)**
dans laquelle $R_2$ a la même signification que dans la formule (I),
ou bien de l'anhydride d'acide correspondant,
préférentiellement en présence de chlorure d'aluminium dans le diméthylformamide, pour obtenir un dérivé de formule générale (V) :

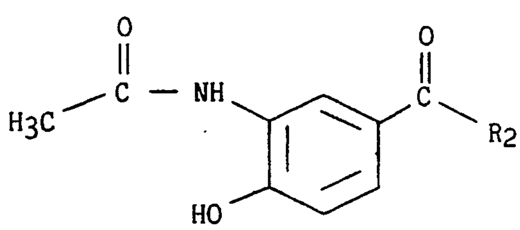

(V)

dans laquelle $R_2$ a la même signification que dans la formule (I),
qui est alors hydrolysé préférentiellement en milieu acide à reflux du milieu réactionnel pour conduire après évaporation puis reprise en milieu acide en présence d'urée à une température comprise entre la température ambiante et la température d'ébullition du milieu ou bien soumis à l'action phosgène, à un dérivé de formule générale (I/a) :

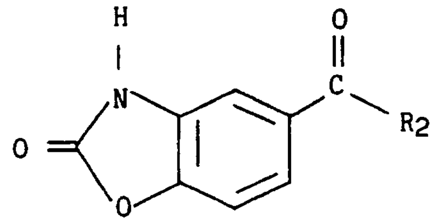

(I/a)

cas particulier des dérivés de formule (I) dans laquelle :
$R_1$ est un atome d'hydrogène,
$R_2$ a la même signification que dans la formule (I),
X et Y représentent ensemble un atome d'oxygène,
qui, si on le souhaite, est soumis en fonction de la structure des dérivés de formule (I) que l'on souhaite obtenir, à l'action d'un agent classique d'alkylation comme par exemple un dialkylsulfate pour conduire à un dérivé de formule (I/b) :

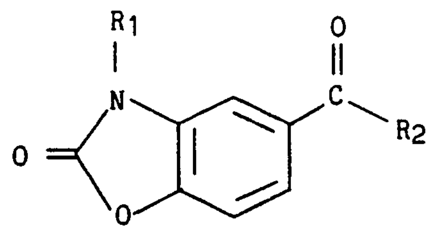

(I/b)

cas particulier des dérivés de formule (I) dans laquelle :
$R_1$ représente un groupement alkyle inférieur,
$R_2$ a la même signification que dans la formule (I),
X et Y représentent ensemble un atome d'oxygène,
dérivé de formule (I/a) ou (I/b), selon le dérivé de formule (I) que l'on souhaite obtenir, qui peut être soumis :
ou bien
à un hydrure mixte de métal alcalin comme par exemple le borohydrure de sodium, préférentiellement en présence d'alcool aliphatique inférieur, pour conduire à un dérivé de formule (I/c) :

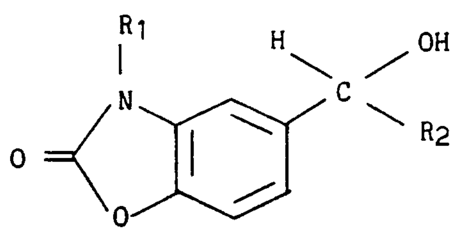

(I/c)

cas particulier des dérivés de formule (I) dans laquelle :
$R_1$ et $R_2$ ont la même signification que dans la formule (I),
X représente un atome d'hydrogène,
Y représente un groupement hydroxyle,
dont on sépare, si on le souhaite, les isomères, par une technique classique de séparation,
ou bien
à une réduction par un trialkylsilane en milieu acide pour conduire à un dérivé de formule (I/d) :

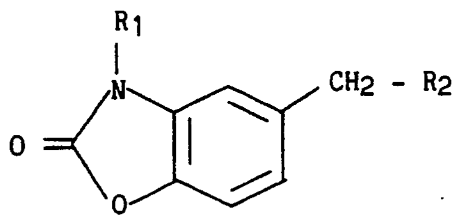

(I/d)

cas particulier des dérivés de formule (I) dans laquelle :
$R_1$ et $R_2$ ont la même signification que dans la formule (I),
X et Y représentent chacun un atome d'hydrogène,
dérivés de formule (I) que l'on purifie, si on le désire, par recristallisation, chromatographie ou toute autre technique classique de purification.

2/ Procédé selon la revendication 1, de préparation de composés de formule (I) dans lesquels X et Y représentent ensemble un atome d'oxygène.

3/ Procédé selon la revendication 1, de préparation de composés de formule (I) dans lesquels $R_1$ représente un atome d'hydrogène.

4/ Procédé selon la revendication 1, de préparation de composés de formule (I) dans lesquels $R_1$ représente un groupement méthyle.

5/ Procédé selon la revendication 1, de préparation de composés de formule (I) dans lesquels $R_2$ représente un groupement aryle.

6/ Procédé selon la revendication 1 de préparation du composé de formule (I) qui est la benzoyl-5 benzoxazolinone.

7/ Procédé selon la revendication 1 de préparation du composé de formule (I) qui est la méthyl-3 benzoyl-5 benzoxazolinone.

Office européen des brevets

# RAPPORT DE RECHERCHE EUROPEENNE

Numero de la demande

EP 90 40 0834

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-3 161 649 (P.J. BREIVOGEL)<br>* En entier * | 1-3,9,<br>10 | C 07 D 263/58<br>A 61 K 31/42<br>C 07 D 413/06 |
| X | CHEMICAL ABSTRACTS, vol. 87, no. 19, 7 novembre 1977, page 593, résumé no. 152172s, Columbus, Ohio, US; & PL-A-85 784 (INSTYTUT PRZEMYSLU FARMACEUTYCZNEGO) 30-07-1976<br>* Résumé * | 1-3 | |
| D,A | FR-A-2 244 506 (INSTITUT NATIONAL DE LA SANTE ET DE LA RECHERCHE MEDICALE)<br>* Revendications * | 1-3,9,<br>10 | |
| A | FR-A-2 244 507 (INSTITUT NATIONAL DE LA SANTE ET DE LA RECHERCHE MEDICALE)<br>* Revendications * | 1-3,9,<br>10 | |
| A | FR-A-2 409 991 (INSTITUT NATIONAL DE LA SANTE ET DE LA RECHERCHE MEDICALE)<br>* Revendications * | 1-3,9,<br>10 | |

| DOMAINES TECHNIQUES RECHERCHES (Int. Cl.5) |
|---|
| C 07 D 263/00<br>A 61 K 31/00<br>C 07 D 413/00 |

Le présent rapport a été établi pour toutes les revendications

| Lieu de la recherche | Date d'achèvement de la recherche | Examinateur |
|---|---|---|
| LA HAYE | 19-06-1990 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)